# EUROPEAN PATENT APPLICATION

(11) **EP 4 360 635 A1**
(43) Date of publication of application: **01.05.2024**
(21) Application number: 22827659.8
(22) Date of filing: 23.06.2022
(51) Int. Cl.: A61K 31/519, C07D 475/04, A61P 27/02

(54) **APPLICATION OF FOLIC ACID DERIVATIVES IN PREPARATION OF DRUGS FOR TREATING CONTACT LENS DISCOMFORT AND XEROPHTHALMIA**

(30) Priority: 24.06.2021 CN 202110704056
(71) Applicant: Lianyungang Jinkang Hexin Pharmaceutical Co. Ltd., Lianyungang, Jiangsu 222000 (CN)
(72) Inventor: CHENG, Yongzhi, Lianyungang, Jiangsu 222000 (CN); GU, Rui, Lianyungang, Jiangsu 222000 (CN); LIAN, Zenglin, Lianyungang, Jiangsu 222000 (CN)
(74) Representative: karo IP
(86) International application number: PCT/CN2022/100814
(87) International publication number: WO 2022/268167

(57) **Abstract**

The present invention relates to a drug for treating dry eye, a method for improving wearing experience in populations with contact lens discomfort, and further to eye drops for treatment.

## Description

### TECHNICAL FIELD

The present invention relates to a drug or health-care food for treating dry eye, a method for improving contact lens wearing experience in a wearer with contact lens discomfort, and further to eye drops for treating dry eye and eye drops for treating symptoms of contact lens discomfort.

### BACKGROUND

When wearing contact lens, the contact lenses will directly interact with the ocular surface and the inner areas of the upper and lower eyelids. The sensory fibers of the trigeminal nerve are densely distributed in these areas. Every year, contact lens wearers give up wearing contact lenses, mainly due to the discomfort during wearing. The contact lens discomfort is characterized by paroxysmal or persistent discomfort of the eyes related to the wearing of contact lenses. At present, although there are limited research evidences on the mechanism of causes of this discomfort reaction, it is worth noting that the symptoms of the symptomatic contact lens wearers (for example, eye irritation, burning sensation, prick and pain) are highly similar to those related to dry eye, which involves inflammatory reaction and neuropathic pain.

Dry eye is a chronic inflammatory disease. The tear instability is accompanied by the increase of tear osmotic pressure, which activates the stress signal in the resident immune cells in the ocular surface epithelium, and triggers the production of congenital inflammatory molecules. The further expression of Th1 cytokine, that is, interferon γ (IFN-γ), causes the dysfunction and death of conjunctival goblet cells, aggravating the instability of tear film, exacerbating the inflammation and forming a vicious circle [Pflugfelder SC, de Paiva CS. The Pathophysiology of Dry Eye Disease: What We Know and Future Directions for Research. Ophthalmology. 2017;124(11S):S4-S13.]. Although great progress is achieved in insight into the pathophysiology of dry eye, the fact is that the pathological mechanism of dry eye cannot be simply attributed to a single cause, and the existing treatment method cannot effectively improve the discomfort and corneal epithelial diseases in all patients.

The word "dry eye" accurately describes a sensation of the eyes. The ocular surface disease index (OSDI) is the most commonly used questionnaire in the clinical diagnosis of dry eye, which is mainly used to diagnose dry eye and evaluate the severity of the disease. OSDI mainly involves the sensation of eyes, including the stimulation of light and wind on eyes, the influence of reading, writing and watching TV on eye comfort, and so on. The above problems are directed to the unpleasant feelings and emotional experiences of the eyes. Without the diagnosis of biomarkers such as inflammatory factors, dry eye can actually be considered as a painful disease. The painful diseases can be roughly divided into nociceptive pain and neuropathic pain, both of which may be related to dry eye. The nociceptive pain is usually short and a result of tissue injury and inflammation. The neuropathic pain is caused by pathological changes or diseases directly affecting the somatosensory system, and generally chronic. In dry eye, the inducements of nociceptive and neuropathic pain may be the same, and these inducement stimuli can lead to temporary or continuous changes in the phenotype of sensory neurons [Galor A, Levitt RC, Felix ER, Martin ER, Sarantopoulos CD. Neuropathic ocular pain: an important yet underevaluated feature of dry eye. Eye (Lond). 2015;29(3):301-312].

The diagnostic definition of dry eye is wide. In practice, patients who visit ophthalmic outpatient department are generally patients with serious symptoms of dry eye, that is, aqueous tear-deficient dry eye, including lacrimal gland deficiency or lacrimal duct obstruction. The dry eye in some patients without tear deficiency is evaporative dry eye. Such patients are more sensitive to wind and light [Chhadva P, Lee T, Sarantopoulos CD, et al. Human Tear Serotonin Levels Correlate with Symptoms and Signs of Dry Eye. Ophthalmology. 2015;122(8):1675-1680]. The above-mentioned dry eye patients with normal tear secretion have highly similar symptoms with symptomatic contact lens wearers, and the pathological mechanisms may also be highly similar. Both of them are very sensitive to cold and hot stimuli [Situ P, Simpson T, Begley C. Hypersensitivity to Cold Stimuli in Symptomatic Contact Lens Wearers. Optom Vis Sci. 2016;93(8):909-916.], and have seasonality. It is generally accepted that the occurrence of contact lens-related conjunctivitis is affected by the time of the year, and the risk of corneal infiltration in winter is increased by 2 to 4 times.

The dry eye in patients with normal tear secretion and symptomatic contact lens wearers is not caused by infection or tissue damage, and is in a state between the inflammation and normal states. Ruslan Medzhitov introduced the concept of "parainflammation". The subtle tissue disturbance caused by contact lenses leads to tissue pressure or failure, which in turn causes parainflammation close to the fundamental state. Under this condition, the resident immune cells will deliver other white blood cells and plasma proteins in a small range according to the severity of the problem, without manifesting the typical characteristics of inflammation [Medzhitov R. Origin and physiological roles of inflammation[J]. Nature, 2008, 454(7203): 428-435]. Although the discomfort in symptomatic contact lens wearers and dry eye patients with normal tear secretion will be alleviated or eliminated when they stop wearing contact lenses or leave the light/wind environment, great pain is still brought to these patients. Because the nerve density in the eye is very high and the secondary nerve is located in the trigeminal ganglion, patients who suffer from pain may be accompanied by migraine or depression, which will have a negative impact on the social, physical and psychological functions.

At present, the treatments of dry eye are mainly directed against inflammation, such as cyclosporine and Lifitegrast targeting T cells that are a key pathophysiological factor leading to chronic dry eye. Lifitegrast is a small molecule, which can inhibit the binding of leukocyte function-associated antigen-1 (LFA-1) on T cells with its ligand intercellular cell adhesion molecule-1 (ICAM1) on the antigen presenting, epithelial and vascular endothelial cells. These molecules improve the symptoms of dry eye in clinical trials, but these treatments can not solve the acute impact of dry eye on the eye surface, and cannot effectively relieve the eye irritation especially under the acute stimulation of light and wind. Corticosteroids are effective in dealing with acute irritation. However, the long-term use of corticosteroids will cause the risks of cataracts and glaucoma.

The treatment against inflammation can not effectively improve the discomfort and corneal epithelial disease in all patients. These patients have neuropathic pain and signs of central sensitization, especially dry eye patients with normal tear secretion and contact lens discomfort. At present, there is also great disputation about the treatment of neuropathic pain. Gabapentin and pentabarine, γ-aminobutyric acid analogues, have been used to suppress abnormal eye sensation, but at present they are only used to relieve severe eye pain. Although these antiepileptic drugs may be helpful to patients with moderate eye discomfort, they lack clinical data, and may have obvious adverse effects. Nerve growth factor (NGF) is an effective stimulator of axon growth and regeneration. NGF is proved to be helpful in the healing of persistent epithelial defects. However, during nerve injury, the release of NGF will increase the neuronal excitability and reduce the pain threshold. Many evidences show that NGF plays an important role in the mediation and enhancement of pain, leading to the development of NGF antagonists as potential analgesics and anti-hyperalgesia drugs. Therefore, whether the NGF antagonist or NGF treatment is beneficial to patients with dry eye still needs further study.

5-methylterahydrofolic acid is a form of folate in human body. Studies have shown that folic acid can reduce the level of homocysteine that is an important risk factor for macular degeneration in the elderly. Studies have shown that combined therapy of folic acid, vitamin B6, and vitamin B12 can reduce the risk of macular degeneration in the elderly. Moreover, folic acid can facilitate the secretion of nerve growth factor to promote the repair of nerve injury. For patients with dry eye, the secretion of NGF may enhance the pain and promote the nerve sensitization. Particularly, NGF promotes the sensitization of non-selective cation channel TRPV1 in injured sensory fibers [Stratiievska A, Nelson S, Senning EN, Lautz JD, Smith SE, Gordon SE. Reciprocal regulation among TRPV1 channels and phosphoinositide 3-kinase in response to nerve growth factor. Elife. 2018;7:e38869.], and The changes in TRPV1 expression are related to the development of thermal hyperalgesia and hyperalgesia. Folinic Acid is also a derivative of folate, which can be converted into 5-methylterahydrofolic acid in the body. At present, there is no research report on the beneficial effect of folic acid on patients with dry eye or contact lens discomfort.

Although many new treatments for dry eye have been developed, drugs approved by drug administration are still insufficient. The main reason for the failure of intervention trial in the treatment of dry eye is the lack of appropriate disease indicators and appropriate preclinical pathological models.

The difficulties in the development of animal model of contact lens discomfort lie in the lack of contact lens design suitable for animals, the challenge in the manufacturing of contact lenses for animals, and the difficulty in guaranteeing the animal compliance. The inventors found that the symptoms of contact lens discomfort are very similar to those of dry eye in some cases. Particularly, patients who have normal tear secretion but feel uncomfortable with dry eyes are usually equally uncomfortable with contact lenses. Recent studies have identified a significant correlation between contact lenses and dry eye [Tan L L, Morgan P, Cai Z Q, et al. Prevalence of and risk factors for symptomatic dry eye disease in S ingapore[J]. Clinical and Experimental Optometry, 2015, 98(1): 45-53]. Therefore, it is possible to use the dry eye model to replace the animal model of contact lens discomfort.

### SUMMARY

A technical solution of the present invention is to provide a new use of a folate derivative. Another technical solution of the present invention is to provide a drug or health-care food comprising the compound for treating dry eye and contact lens discomfort symptoms, and provide an eye drop for treating dry eye and contact lens discomfort symptoms.

Use of a compound of general Formula I of the present invention in the preparation of a drug or health-care food for treating dry eye or alleviating contact lens discomfort is provided, 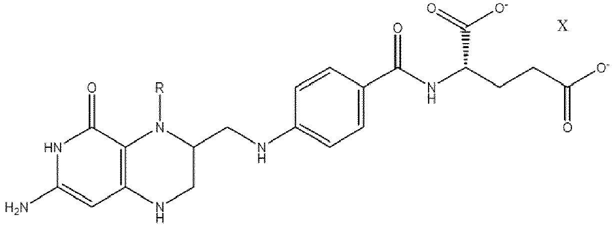 where R is selected from methyl or formyl; and X is a substance capable of forming a pharmaceutically acceptable salt with 5-methylterahydrofolic acid or folinic acid, including calcium ion, sodium ion, an organic amine, and glucosamine.

The dry eye is evaporative dry eye that has normal lacrimal gland secretion, but is sensitive to external stimuli, and particularly has the symptoms of anemophobia, photophobia, eye dryness, and burning sensation. The symptoms of contact lens discomfort comprise eye dryness, pain, foreign body sensation, itching, and other symptoms.

The present invention also provides an eye drop for treating dry eye, which comprises 5-methylterahydrofolic acid or a pharmaceutically acceptable salt thereof, and is used for inhibiting eye discomfort caused by light or wind. Preferably, the eye drop for treating dry eye provided in the present invention also comprises arginine.

Preferably, 5-methylterahydrofolic acid or a pharmaceutically acceptable salt thereof is comprised in a concentration of 0.01-10 wt/vol%.

The present invention further provides a drug or health-care food for patients with contact lens discomfort, which comprises 5-methylterahydrofolic acid or a pharmaceutically acceptable salt thereof, and is used to relieve dryness, gritty feeling, itching and other symptoms. The drug or health-care food for patients with contact lens discomfort comprises an oral preparation, and eye drops.

As disclosed herein, based on the finding that 5-methylterahydrofolic acid can improve the contact lens wearing experience of symptomatic contact lens wearers, the present invention provides a method to take positive measures to improve the contact lens wearing experience of these patients. In addition, the pathological mechanism in symptomatic contact lens wearers is similar to that in some patients with dry eye, especially those with dry eye and normal lacrimal gland secretion. 5-methylterahydrofolic acid can also improve the eye experience of this type of patients. 5-methylterahydrofolic acid can reduce the expression of some inflammatory factors, particularly, IL-17, in ocular surface tissues, and inhibit the expression of MMP-2 in ipsilateral trigeminal ganglion of the eye.

The research of the present invention shows that the mechanism of chronic dry eye, particularly dry eye with normal lacrimal gland secretion, but dry eye symptoms may be different from that of severe dry eye. The present invention finds that after mice are stimulated by acute dry wind and cultured in normal environment for 3 months, the cornea of the eyes of the mice is still damaged. However, the expression of inflammatory factors in ocular surface tissues is different from that in many previous studies. No high-level expressions of inflammatory factors and MMP-9 are observed, and chronic dry eye involves persistent ocular surface inflammation related to Th17 reaction, which may be mainly or solely mediated by Th17 cells. With regard to the further phenotype of T cells after the occurrence of inflammation, Th17 shows the remarkable property of long-effect memory cells. The present invention finds that chronic dry eye involves not only inflammation of the ocular surface but also chronic neuroinflammation. The present invention discloses for the first time some specific effects of chronic dry eye syndrome on ocular nerves, particularly, MMP-2.

A recent study shows that the level of urea in tear film may be a potential diagnostic marker of dry eye. In populations with dry eye, the level of urea in tear film is significantly lower than that in healthy people. Urea is formed during the metabolism of some amino acids, especially arginine. The present invention finds that arginine alone can not significantly improve the ocular surface damage of model mice, but it has a significantly improved performance after being used in combination with folinic acid.

### BRIEF DESCRIPTION OF THE DRAWINGS

Fig. 1 shows IL-17 contents in tears of various contact lens wearer (*, p < 0.05 compared with group C; #, p < 0.05 compared with group B).
Fig. 2 shows the tear secretion test in mice model of dry eye (*, P < 0.05 compared with the control group).
Fig. 3 shows the corneal staining test in dry eye model of mice (*, P < 0.05 compared with the control group).
Fig. 4 investigates the effect of 5-methylterahydrofolic acid eye drops on tear secretion in mice.
Fig. 5 investigates the effect of 5-methylterahydrofolic acid eye drops on ocular surface damage of mice (*, P < 0.05 compared with the control group).
Fig. 6 investigates the effect of 5-methylterahydrofolic acid eye drops on inflammatory factors on ocular surface of mice (*, P < 0.05 compared with the control group).
Fig. 7 investigates the effect of 5-methylterahydrofolic acid eye drops on the expression of metalloproteinases and inflammatory factors in trigeminal ganglion of mice (*, P < 0.05 compared with the control group).
Fig. 8 investigates the effect of eye drops of folinic acid, folinic acid and arginine in combination, and 5-methylterahydrofolic acid on the tear secretion in mice.
Fig. 9 investigates the effect of eye drops of folinic acid, folinic acid and arginine in combination, and 5-methylterahydrofolic acid on ocular surface damage of mice (*, P < 0.05 compared with the control group).

### DETAILED DESCRIPTION

### Example 1. Preliminary investigation on improvement of symptoms of contact lens discomfort

A total of 10 adults suffering from contact lens discomfort were included in the study. Among them, 8 felt uncomfortable when wearing contact lenses, and 2 were asymptomatic contact lens wearers. The contact lenses worn by the participants were all silicone hydrogelbased contact lenses. All the participants were in good health. The symptoms and severity of eye discomfort were evaluated by the ocular surface disease index (OSDI), and whether there were symptoms such as inflammation in the eyes was checked by an optometrist.

The average age of the participants was 33 years, and the standard deviation SD was 11 years. 3 males and 7 females were included. It was found that all participants had no typical ocular inflammation. Compared with asymptomatic contact lens wearers, the symptomatic wearers were more sensitive to light and wind (n=6, 75%).

The symptomatic contact lens wearers were divided into 2 groups, that is, group A (n=4) and group B (n=4). The asymptomatic contact lens wearers were assigned to group C The participants in each group didn't wear contact lenses for 24 hours, and then the tears were collected. Dietary supplements containing 5 mg calcium salt of 6S-5-methylterahydrofolic acid/tablet were orally taken by the participants in group A every day. After 3 days, the participants in each group began to wear contact lenses, and the tears were collected after 6 hrs. The contents of IL-2, IL-6, IL-17, and TNF-α were detected (ELISA method). The participants in each group were followed up to determine the degree of discomfort of wearing contact lenses.

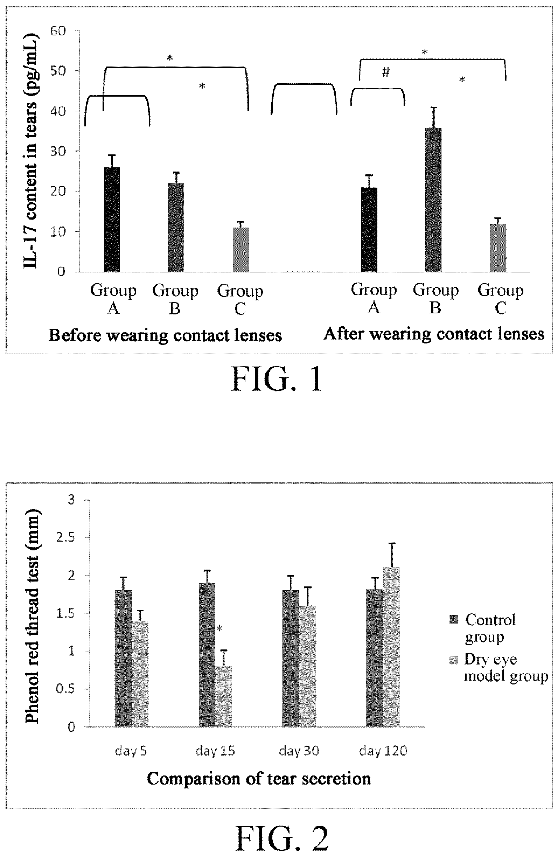

Statistical analysis: 3 participants in group A said that the contact lens discomfort almost disappears, and only 1 case thinks that the effect is not significant. It is difficult to detect and quantify the difference between various groups with respect to the inflammatory factors. However, the difference in IL-17 level is shown between people with contact lens discomfort and normal people. 5-methylterahydrofolic acid can reduce the level of IL-17 in tears, as shown in Fig. 1.

### Example 2. Construction of mouse model of chronic dry eye

Dry eye includes aqueous-deficient dry eye and evaporative dry eye. The different developments of typical dry eyes are pointed out [Bron AJ, Yokoi N, Gafney E, Tiffany JM. Predicted phenotypes of dry eye: proposed consequences of its natural history. Ocul Surf. 2009;7(2):78-92]. In the development of evaporative dry eye, the integrity of the tear film lipid layer decrease, and the evaporation of tears increases, causing the increase of osmotic pressure. Thus, the corneal nerve endings are stimulated, and dry eye symptoms occur, causing increased blinking and compensatory increase in tear secretion. The exact mechanism of ocular surface changes affecting the tear function is not clear. However, in the development of some patients with dry eye, tear secretion does not decrease, but increases. Similar obvious contradictory results have been proved in human clinical observation.

It can be seen that a suitable animal model of dry eye is very important, and it is impossible to show the true state of dry eye or contact lens discomfort only by destroying the lacrimal gland or modeling with hypertonic saline. The mouse model of dry eye constructed under specific environmental conditions is closest to the real situation.

120 female BALB/c mice aged 8-12 weeks were placed in an isolated and sealed cage with an opening on the top. Air dried through anhydrous CaSO₄ was pumped into the cage by an air pump, where the air humidity RH = 19.5% ± 5%, the flow rate was 15 L/min, and the temperature was 21-23°C. The mice in the dry and windy environment were taken out on the 5th, 15th and 30th day respectively. The production of tears was measured by phenol red thread test, and compared with that of the mice raised in standard cages in normal environment. Meanwhile, the cornea of the mice were stained with fluorescein, and the punctate staining was recorded in the cornea.

The results show that the tear secretion of mice decreases on the fifth day in the dry environment, but increases on the 30th day, indicating that compensatory tear increase may occur. After 30 days, the mice in dry environment were transferred to a standard feeding box (temperature: about 20°C, and humidity: 40-60%), and raised for 3 months. The mice were taken out for tear secretion measurement and corneal fluorescence staining test. The results show that the tear secretion of the mice returned to normal or even supranormal level (see Fig. 2).

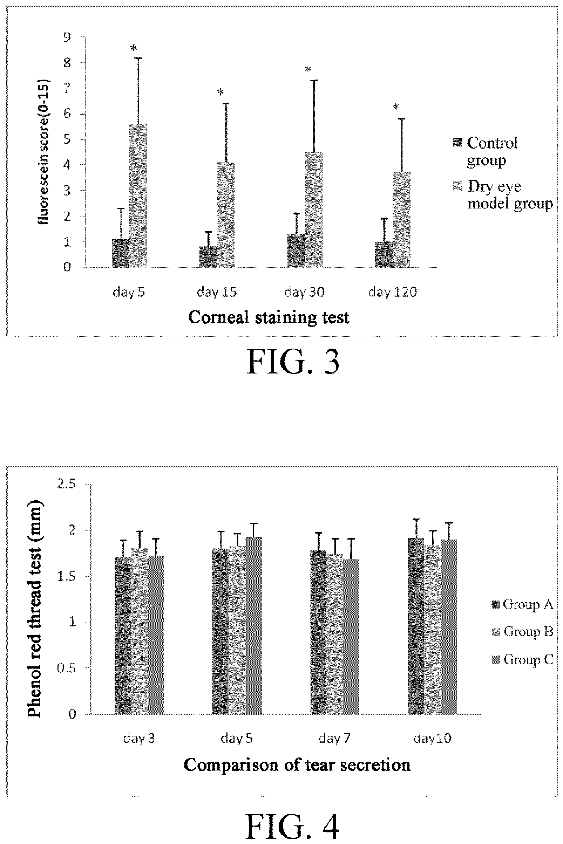

The corneal staining test showed that the corneal injury in mice does not disappear with the recovery of tear secretion, and the mice show persistent low-level corneal injury (see Fig. 3).

### Example 3. Effect of 5-methylterahydrofolic acid on model mice of choronic dry eye

5-methylterahydrofolic acid eye drops were prepared as follows. Freeze-dried powder of arginine 6S-5-methylterahydrofolate was mixed with purified water to prepare a
0.5% 5-methylterahydrofolic acid eye drop. Arginine hydrochloride was mixed with purified water to give a concentration of 0.5%.

The experiment was divided into 3 groups. 20 mice with chronic dry eye in Example 2 were divided into a 5-methylterahydrofolic acid group (group A, n=10), and an arginine group (group B, n=10). 4 normal mice were taken as the control group. Each eye of the mice in group A was dripped with 5 µL of 5-methylterahydrofolic acid eye drop every day, each eye of the mice in group B was dripped with 5 µL of 0.5% arginine eye drop every day, and each eye of the mice in group C was dripped with 5 µL of a phosphate buffer every day. Tears were collected on the 3rd, 5th, 7th and 10th day after treatment, and the secretion of tears was detected. On the 10th day after treatment, the cornea of the mice in each group were stained with fluorescein.

After treatment, the animals were euthanized, and the eye tissues such as cornea, lacrimal gland and meibomian gland were taken out and washed in PBS. The cornea, conjunctiva and draining lymph node were collected and stored in cold sterile PBS at -80°C. The samples were homogenized and centrifuged on ice. The levels of IFN-γ, IL-17 and IL-6 in the supernatant were determined by ELISA kit (Raybiotech).

The ipsilateral trigeminal ganglion and trigeminal trunk of the eye of the mice were dissected quickly on ice, immediately frozen with liquid nitrogen, and stored at -80°C. RNA was extracted from ipsilateral trigeminal ganglion and trigeminal trunk by using NucleoSpin RNA purification II kit (NucleoSpin RNAS, Germany), and PCR amplification was carried out with mouse-specific cDNAs for matrix metalloproteinases MMP-9, MMP-2, and TNF-α. For each primer, the reaction was stopped after 24, 28, 32, 36, or 40 cycles, so as to ensure that the PCR products were within the linear range of the amplification curve, and semi-quantitative RT-PCR for the expression of related genes was realized.

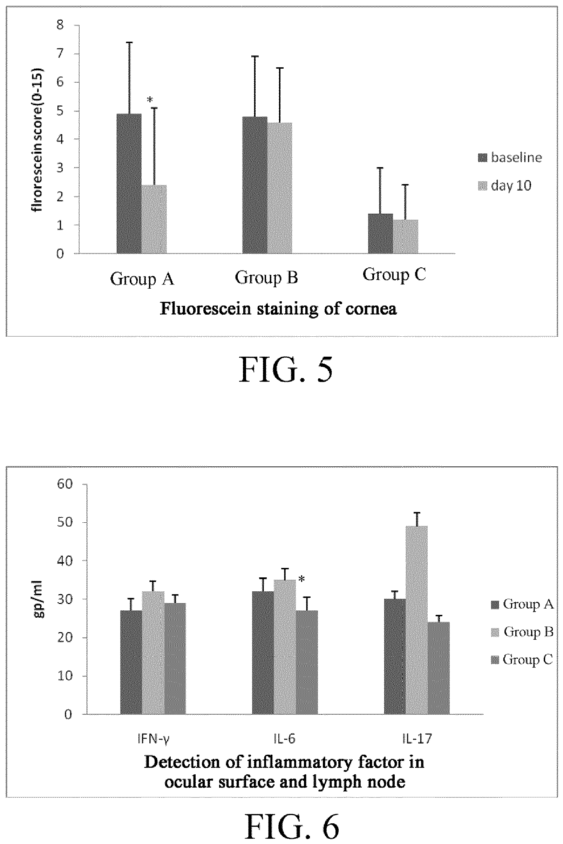

The results show that there was no significant difference in tear secretion in the eyes of mice in each group (see Fig. 4). The fluorescein staining shows that 5-methylterahydrofolic acid can improve the corneal injury with a significant difference from the baseline state (see Fig. 5). The results from the ocular surface and lymph node show that the inflammatory factors of chronic dry eye in this model are not induced to express. This may be attributed to the fact that the immune cells residing on the ocular surface can show significant differences only after receiving stimulation. However, IL-17 shows great difference (see Fig. 6), which may be due to the fact that Th17 cells expressing IL-17 reside in the ocular surface of chronic dry eye. The present invention finds that 5-methylterahydrofolic acid eye drops can significantly reduce IL-17 inflammatory factor, which is consistent with the above experimental results.

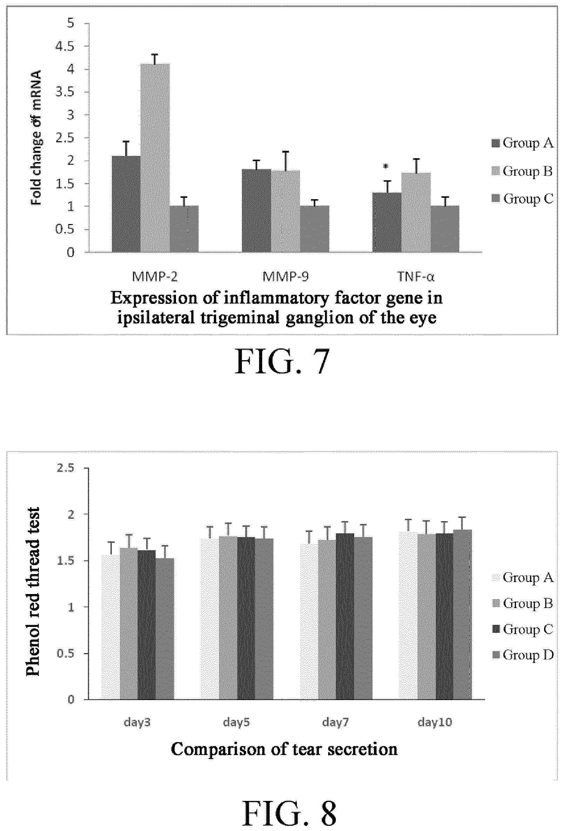

In the present invention, the expression of relevant inflammatory genes in trigeminal nerve in dry eye is also investigated (see Fig. 7). The result shows that 5-methylterahydrofolic acid eye drops can significantly inhibit the MMP-2 gene expression, and also have a certain inhibitory effect on TNF-α, but not on MMP-9 significantly. It has been reported that the expression of metalloproteinases in nerve cells can mediate nerve pain. MMP-9 mediates the earlier pain during injury, and MMP-2 tends to mediate chronic pain. The results are unexpected. It may be because 5-methylterahydrofolic acid can penetrate the aqueous circulation barrier and directly act on the nervous system.

### Example 4. Effect of folinic acid on model mice of chronic dry eye

The stability of arginine 5-methylterahydrofolate in water is very poor. Therefore, it is necessary to verify the therapeutic potential of levofolinic acid in model mice of dry eye. 100 mg of cacium levofolinate was dissolved in 20 mL of purified water to prepare a calcium folinate eye drop. Freeze-dried powder of arginine 6S-5-methylterahydrofolate was mixed with purified water to prepare a 0.5% eye drop. 100 mg of calcium levofolinate, 80 mg of arginine hydrochloride and 20 ml of purified water were prepared into a combined eye drop.

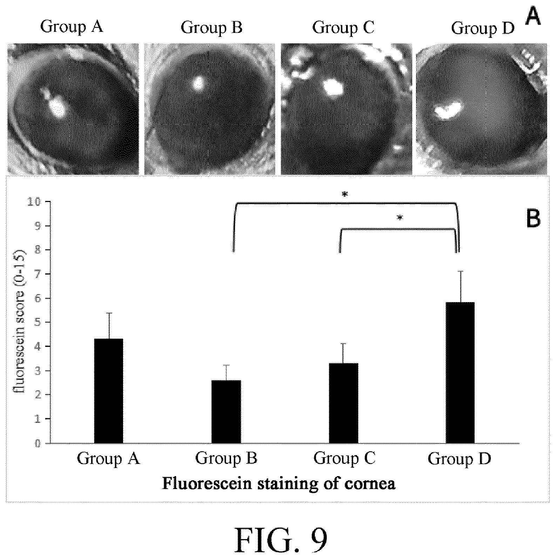

The experiment was divided into 4 groups. 40 mice with chronic dry eye in Example 2 were assigned to a levofolinic acid group (group A, n=10), a 5-methylterahydrofolic acid group (group B, n=10), a calcium levofolinate + arginine group (group C, n=10), and a phosphate buffer (group D) n=10) as control group. Tears were collected on the 3rd, 5th, 7th and 10th day after treatment, and the secretion of tears was detected. On the 10th day after treatment, the cornea of the mice in each group were stained with fluorescein. The results show that there is no significant difference in tear secretion in the eyes of mice in each group (see Fig. 8). The fluorescein staining shows that the combination of calcium levofolinate and arginine, and arginine 5-methylterahydrofolate can improve the corneal injury, with a significant difference from the baseline state (see Fig. 9). The data shows that the combined use of calcium levofolinate and arginine be a new method to relieve dry eye.

## Claims

1. Use of a compound of general Formula I in the preparation of a drug or health-care food for treating dry eye or contact lens discomfort, 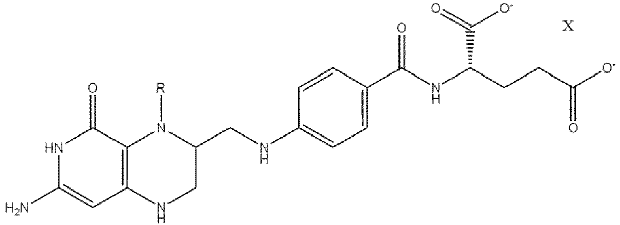 wherein R is selected from methyl or formyl, X is a substance capable of forming a pharmaceutically acceptable salt with 5-methylterahydrofolic acid or folinic acid, including calcium ion, sodium ion, an organic amine, and glucosamine.

2. The use according to claim 1, wherein the dry eye is evaporative dry eye.

3. The use according to claims 1 and 2, wherein the drug comprises eye drops for treating dry eye.

4. The use according to claim 3, wherein the eye drops comprise arginine.

5. The use according to claim 4, wherein the eye drops inhibit inflammation caused by the expression of inflammatory factors on the ocular surface, and inhibit neuritis and neuralgia caused by the expression of metalloprotease MMP-2 and TNF-α in trigeminal ganglion of eyes.

6. The use according to claim 1, wherein the symptoms of contact lens discomfort comprise eye dryness, pain, foreign body sensation, and itching.

7. The use according to claim 6, wherein the drug or health-care food inhibits inflammation caused by the expression of inflammatory factors on the ocular surface, and inhibits neuritis and neuralgia caused by the expression of metalloprotease MMP-2 and TNF-α in trigeminal ganglion of eyes.

8. The use according to any one of claims 4 to 7, wherein the eye drops for treatment also comprises a pharmaceutically acceptable auxiliary material.
